# EUROPEAN PATENT SPECIFICATION

(11) **EP 0 861 106 B1**
(45) Date of publication and mention of the grant of the patent: **28.07.1999**
(21) Application number: 96933346.7
(22) Date of filing: 20.09.1996
(51) Int. Cl.: A61M 27/00

(54) **DEVICE FOR DRAINING THE PLEURAL CAVITY**
DRAINAGEVORRICHTUNG FÜR DIE PLEURAHÖHLE
DISPOSITIF DE DRAINAGE DE LA CAVITE PLEURALE

(30) Priority: 22.09.1995 DE 19535165
(43) Date of publication of application: 02.09.1998
(73) Proprietor: Jakob, Heinz, 69126 Heidelberg (DE)
(72) Inventor: Jakob, Heinz, 69126 Heidelberg (DE)
(74) Representative: Patentanwälte Sternagel & Fleischer
(86) International application number: EP9604122
(87) International publication number: WO9710872

(56) References cited:
- US-A- 3 960 153
- US-A- 4 221 220
- US-A- 4 382 442
- US-A- 4 512 765

## Description

The present invention relates to a device for draining the pleural cavity.

Heart surgery quite often entails opening the pleural cavity. In particular, the left pleural cavity is almost always opened when preparing the internal thoracic artery, and this means that the corresponding pleural cavity has to be provided with a drainage system. This drainage system is typically introduced into the pleural cavity through an intercostal incision. Since the pleura, which is provided with a dense network of sensitive nerve endings, is thereby punctured and acquires a chronic pain impulse, this phase generally causes great discomfort to patients. This discomfort can be alleviated by analgesic medication, but it can never be completely eliminated. In order to eliminate these sensations of pain, or at least to substantially reduce them, it has been proposed to insert the pleural drainage system through the opening which has been made anyway in the median pleura, thereby avoiding the intercostal spaces. To do this, the drainage system, as is customary in heart surgery, is introduced through the rectus fascia just to the side of the midline and then inserted to the left or right through the opened pleura into the pleural space. It has now been found that the straight thorax drainage systems which have hitherto been used do not remain lying in the sulcus phrenico costalis, as they are intended to do, but instead right themselves and so do not remain placed at the deepest point of the pleural cavity. It is thus no longer possible to ensure that the pleural cavity is drained as completely as possible.

US-A-4382442 discloses a thoracostomy tube which drains fluid from the human body comprising a tubular middle part as well as tubular proximal and distal end parts which together form a flexible drainage tube which is open at both ends and has one lateral opening in the distal end part at a distance from the end opening whereby the proximal and distal end parts are angled off from the middle part.

From US-A-4512765 a tracheal bronchial catheter is known that is provided with a double bend at the distal end.

The object of the present invention is now to remedy these disadvantages of the known drainage devices.

This object is achieved by a device for draining the pleural cavity, comprising a tubular middle part (1), a tubular distal end part (2), and a tubular proximal end part (3), which together form a flexible drainage tube which is open at both ends (4,5), at least one lateral opening (6) being provided in the tube wall in the distal end part (2) at a distance from the end opening (4), the proximal end part (3) and distal end part (2) being angled off from the middle part (1), characterized in that the proximal end part (3) and the distal end part (2) are angled off in the same direction to one another, whereby an angle of between 95° and 145° is included between the middle part (1) and the distal end part (2), and an angle of between 65° and 115° is included between the middle part (1) and the proximal end part (3).

Preferred embodiments of this device are described in the subclaims.

The drainage device according to the invention can be inserted via a median incision in the rectus fascia, so that it can then be placed reliably in the costophrenic angle in accordance with the two pre-set angles. This ensures that the drainage device does not right itself, and instead remains at all times at the deepest point of the pleural cavity.

According to the present invention an angle of between 95° and 145° is included between the middle part and the distal end part, and an angle of between 65° and 115° is included between the middle part and the proximal end part. In a particularly preferred embodiment, the angle between the middle part and the distal end part is 120° and the angle between the middle part and the proximal end part is 90°.

In order to ensure the best possible drainage of the pleural cavity, at least one lateral opening, preferably three lateral openings, is/are provided in the tube wall in the distal end part at a distance from the end opening, In the preferred embodiment, the three lateral openings are arranged equidistant from one another, it being particularly advantageous if the lateral openings are provided in a spiral formation, offset 120° in relation to one another, in the outer wall of the distal end part of the drainage tube. The distal end of the drainage tube can be rounded in order to facilitate the insertion of the drainage tube.

It is also advantageous, starting from the proximal lateral opening, to provide a measurement scale which extends from the distal end part via the middle part as far as the proximal end part.

The drainage tube preferably consists of a flexible, transparent, medically compatible plastic. One example of the latter is PVC. In order to be able to check the positioning of the drainage tube during surgery, an X-ray contrast strip is provided along the length of the drainage tube. The width of this contrast strip can be 1 to 3 mm. Suitable X-ray contrast media are those known to the person skilled in the art, such as, for example, barium sulphate.

In order to facilitate the attachment of the drainage tube to the corresponding connectors, the cross-section of the proximal end part widens conically towards the proximal end opening in an end portion. This end portion can have a length of 55 ± 10 mm. It is particularly advantageous in this respect if the proximal end opening of the drainage tube is preferably bevelled at an angle of 30 ± 15°. The conically widened end portion can additionally be angled off in the direction of the distal end part, in relation to the longitudinal axis of the remaining proximal end part.

Except for the conically widened end portion in the proximal end part, the diameter is essentially constant and can be varied in accordance with the application requirements. For example, diameters of 20 to 32 Charrière units are envisaged. The extended length of the drainage tube can be varied in accordance with the chosen diameter and amounts, for example, to 42 cm for the small diameters and at least 50 cm, preferably 55 cm, for the large diameters. The drainage device according to the invention is intended for one-off use and, accordingly, can be offered, together with accessories, as a sterile disposable article in a customary double-sterile package.

The present invention is now explained in more detail on the basis of the drawing.

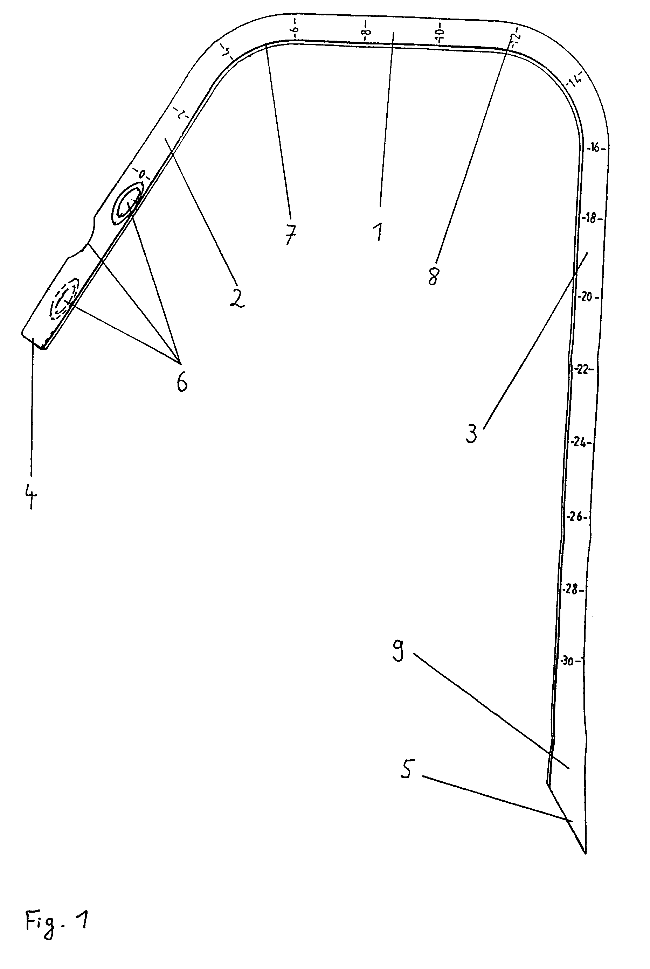

Figure 1 shows a side view of a preferred embodiment of the device according to the invention for draining the pleural cavity.

As can be seen from Figure 1, the middle part 1 forms an angle of ca. 120° with the distal end part 2 of the drainage tube, and an angle of ca. 90° with the proximal end part 3. In the distal end part 2, at a distance from the distal end opening 4, there are three lateral openings 6 which are equidistant from one another and which are in a spiral formation, in each case offset 120°, in the side wall of the distal end part 2. Starting from the proximal lateral opening, a measurement scale in cm gradations extends from the distal end part 2 via the middle part 1 up to and including the proximal end part 3.

In addition, an X-ray contrast strip 7 is provided along the entire length of the drainage tube.

In the end portion 9 of the proximal end part 3, the otherwise approximately constant diameter of the drainage tube widens continuously, with the proximal end opening 5 having a bevelled design. In addition, the end portion 9 is angled in the direction of the distal end part 2 in relation to the longitudinal axis of the proximal end part 3.

## Claims

1. Device for draining the pleural cavity, comprising a tubular middle part (1), a tubular distal end part (2), and a tubular proximal end part (3), which together form a flexible drainage tube which is open at both ends (4,5), at least one lateral opening (6) being provided in the tube wall in the distal end part (2) at a distance from the end opening (4), the proximal end part (3) and distal end part (2) being angled off from the middle part (1), characterized in that the proximal end part (3) and the distal end part (2) are angled off in the same direction to one another, whereby an angle of between 95° and 145° is included between the middle part (1) and the distal end part (2), and an angle of between 65° and 115° is included between the middle part (1) and the proximal end part (3).

2. Device according to claim 1, characterized in that the angle between the middle part (1) and the distal end part (2) is 120° and the angle between the middle part (1) and the proximal end part (3) is 90°.

3. Device according to one of the preceding claims, characterized in that three lateral openings (6) are provided in the distal end part (2).

4. Device according to claim 3, characterized in that the lateral openings (6) are arranged equidistant from one another.

5. Device according to claim 3 or 4, characterized in that the lateral openings (6) are arranged in a spiral formation, offset 120° to one another.

6. Device according to claim 5, characterized in that, starting from the proximal lateral opening, a measurement scale (8) extends from the distal end part (2) via the middle part (1) up to and including the proximal end part (3).

7. Device according to one of the preceding claims, characterized in that the device consists of a transparent, medically compatible plastic.

8. Device according to one of the preceding claims, characterized in that an X-ray contrast strip (7) is provided along the length of the drainage tube.

9. Device according to one of the preceding claims, characterized in that, in an end portion (9), the diameter of the proximal end part (3) widens continuously towards the proximal end opening (5), and the proximal opening (5) is bevelled.

## Patentansprüche

1. Vorrichtung zur Drainage der Pleurahöhle mit einem rohrförmigen Mittelteil (1), einem rohrförmigen distalen Endteil (2) und einem rohrförmigen proximalen Endteil (3), die gemeinsam ein an beiden Enden (4, 5) offenes, flexibles Drainagerohr ausbilden, wobei im distalen Endteil (2) in Abstand zu der Endöffnung (4) in der Rohrwand mindestens eine Seitenöffnung (6) vorgesehen ist, und das proximale Endteil (3) und das distale Endteil (2) gegenüber dem Mittelteil (1) abgewinkelt sind, dadurch gekennzeichnet, daß das proximale Endteil (3) und das distale Endteil (2) in dieselbe Richtung aufeinander zu abgewinkelt sind, wobei ein Winkel zwischen 95° und 145° zwischen dem Mittelteil (1) und dem distalen Endteil (2) und ein Winkel zwischen 65° und 115° zwischen dem Mittelteil (1) und dem proximalen Endteil (3) eingeschlossen ist.

2. Vorrichtung nach Anspruch 1, dadurch gekennzeichnet, daß der Winkel zwischen dem Mittelteil (1) und dem distalen Endteil (2) 120° und der Winkel zwischen dem Mittelteil (1) und dem proximalen Endteil (3) 90° beträgt.

3. Vorrichtung nach einem der vorhergehenden Ansprüche, dadurch gekennzeichnet, daß in dem distalen Endteil (2) drei Seitenöffnungen (6) vorgesehen sind.

4. Vorrichtung nach Anspruch 3, dadurch gekennzeichnet, daß die Seitenöffnungen (6) äquidistant zueinander angeordnet sind.

5. Vorrichtung nach Anspruch 3 oder 4, dadurch gekennzeichnet, daß die Seitenöffnungen (6) spiralförmig jeweils um 120° verdreht zueinander angeordnet sind.

6. Vorrichtung nach Anspruch 5, dadurch gekennzeichnet, daß ausgehend von der proximalen Seitenöffnung sich eine Meßskala (8) von dem distalen Endteil (2) über das Mittelteil (1) bis einschließlich zu dem proximalen Endteil (3) erstreckt.

7. Vorrichtung nach einem der vorhergehenden Ansprüche, dadurch gekennzeichnet, daß die Vorrichtung aus einem durchsichtigen medizinisch verträglichen Kunststoff besteht.

8. Vorrichtung nach einem der vorhergehenden Ansprüche, dadurch gekennzeichnet, daß entlang der Länge des Drainagerohrs ein Röntgenkontraststreifen (7) vorgesehen ist.

9. Vorrichtung nach einem der vorhergehenden Ansprüche, dadurch gekennzeichnet, daß sich der Querschnitt des proximalen Endteils (3) zu der proximalen Endöffnung (5) hin in einem Endabschnitt (9) kontinuierlich vergrößert und die proximale Öffnung (5) abgeschrägt ist.

## Revendications

1. Dispositif de drainage de la cavité pleurale comprenant une partie intermédiaire tubulaire (1), une partie d'extrémité distale tubulaire (2) et une partie d'extrémité proximale tubulaire (3) qui, ensemble, forment une sonde de drainage souple qui est ouverte aux deux extrémités (4,5), au moins une ouverture latérale (6) étant prévue dans la paroi de la sonde dans la partie d'extrémité distale (2) à distance de l'ouverture d'extrémité (4), la partie d'extrémité proximale (3) et la partie d'extrémité distale (2) formant un angle par rapport à la partie intermédiaire (1), caractérisé en ce que la partie d'extrémité proximale (3) et la partie d'extrémité distale (2) sont toutes deux coudées dans la même direction, de sorte qu'un angle compris entre 95° et 145° est inclus entre la partie intermédiaire (1) et la partie d'extrémité distale (2) et qu'un angle compris entre 65° et 115° est inclus entre la partie intermédiaire (1) et la partie d'extrémité proximale (3).

2. Dispositif selon la revendication 1, caractérisé en ce que l'angle entre la partie intermédiaire (1) et la partie d'extrémité distale (2) est de 120° et l'angle entre la partie intermédiaire (1) et la partie d'extrémité proximale (3) est de 90°.

3. Dispositif selon l'une des revendications précédentes, caractérisé en ce que trois ouvertures latérales (6) sont prévues dans la partie d'extrémité distale (2).

4. Dispositif selon la revendication 3, caractérisé en ce que les ouvertures latérales (6) sont disposées à égale distance les unes des autres.

5. Dispositif selon la revendication 3 ou 4, caractérisé en ce que les ouvertures latérales (6) sont disposées en formation de spirale et décalées de 120° les unes par rapport aux autres.

6. Dispositif selon la revendication 5, caractérisé en ce que, en partant de l'ouverture latérale proximale, une échelle de mesure (8) s'étend de la partie d'extrémité distale (2) via la partie intermédiaire (1) jusqu'à la partie d'extrémité proximale (3) comprise.

7. Dispositif selon l'une des revendications précédentes, caractérisé en ce que le dispositif est en plastique transparent médicalement compatible.

8. Dispositif selon l'une des revendications précédentes, caractérisé en ce qu'une bande de contraste radiographique (7) est prévue sur toute la longueur de la sonde de drainage.

9. Dispositif selon l'une des revendications précédentes, caractérisé en ce que, dans une partie terminale (9), le diamètre de la partie d'extrémité proximale (3) s'élargit en continu vers l'ouverture de l'extrémité proximale (5) et l'ouverture proximale (5) est biseautée.
